Europäisches Patentamt

European Patent Office

Office européen des brevets

(19)

(11) Numéro de publication: **0 479 005 B1**

(12) **FASCICULE DE BREVET EUROPEEN**

(45) Date de publication du fascicule du brevet: **06.12.95**

(51) Int. Cl.6: **A61K 9/00**, A61K 9/12, A61K 9/50, A61K 47/02, A61K 47/10, A61K 47/14, A61K 47/32, A61K 47/36, A61K 47/38

(21) Numéro de dépôt: **91115437.5**

(22) Date de dépôt: **12.09.91**

(54) **Composition pharmaceutique sous forme de gel dans un emballage distributeur.**

(30) Priorité: **04.10.90 EP 90118920**

(43) Date de publication de la demande:
**08.04.92 Bulletin 92/15**

(45) Mention de la délivrance du brevet:
**06.12.95 Bulletin 95/49**

(84) Etats contractants désignés:
**AT BE CH DE DK ES FR GB GR IT LI LU NL SE**

(56) Documents cités:
**EP-A- 0 379 147**
**FR-A- 2 631 831**
**US-A- 4 427 681**
**US-A- 4 576 645**

(73) Titulaire: **SOCIETE DES PRODUITS NESTLE S.A.**
**Case postale 353**
**CH-1800 Vevey (CH)**

(72) Inventeur: **Tachon, Pierre**
**23, Ch.de L'Epi d'Or**
**CH-1023 Cugy (CH)**
Inventeur: **Wagneur, Béatrice**
**4, rue Clouet des Pesruches**
**F-49000 Angers (FR)**
Inventeur: **Viret, Jean-Louis**
**La Bruyère A. Fontanivent**
**F-1817 Brent (FR)**

Rank Xerox (UK) Business Services
(3.10/3.09/3.3.3)

## Description

L'invention a trait à une composition pharmaceutique dans un emballage, dans laquelle le principe actif est présenté dans un excipient sous forme de gel. Depuis plusieurs siècles, les médicaments ont été couramment administrés par voie orale sous forme de sirop dans des flacons ou sous forme de comprimés ou de capsules.

Il s'agit de présentations faciles à absorber et permettant de conserver les substances actives. Cependant, la présentation sous forme de sirop souffre d'un certain nombre d'inconvénients, par exemple :

- le fort apport de sucre peut être néfaste, par exemple chez les diabétiques,
- la dose thérapeutique n'est pas directement accessible, nécessitant l'utilisation d'une mesure, par exemple une cuillère, et de ce fait, la dose administrée n'est pas précise,
- le mode de présentation se prête mal à l'administration d'une dose thérapeutique chez les bébés et nourrissons qui peuvent détourner la bouche de la cuillère ou renverser le sirop,
- les enfants peuvent prendre en une fois la totalité du flacon et risquer de s'intoxiquer.

Par ailleurs, les présentations, sous forme de comprimés ou de capsules ne sont pas adaptées à certains patients, par exemple les petits enfants ou les gériatres ayant des problèmes de déglutition.

EP-A-0 379 147 concerne un gel extrudable comme support de principe actif qui peut être distribué dans un emballage muni d'une pompe doseuse. Le gel en question contient le principe actif en solution et comporte un agent gélifiant à base d'extrait d'algue, par exemple un carrhagénate. Le fait que le principe actif doive être en solution impose une concentration faible, si bien que l'administration d'une dose thérapeutique quotidienne nécessite dans l'exemple donné 12 à 60 pressions réparties en 3 ou 4 prises sur la pompe et consomme le volume de produit d'un emballage entier, ce qui est un énorme inconvénient. Ce défaut ne peut pas être corrigé par une simple augmentation de la concentration du principe actif, car c'est au détriment de la stabilité du gel, la masse étant visqueuse et non gélifiée et le principe actif non solubilisé, ainsi que de son acceptabilité organoleptique.

US-A-4427681 concerne une composition pharmaceutique sous forme de gel thixotrope aisément transformable en liquide versable par agitation. Le but est ici de mettre le principe actif ainsi que l'agent opacifiant dioxyde de titane en suspension stable pendant l'entreposage et de pouvoir distribuer la composition sous forme d'un liquide versable hors d'une bouteille par simple agitation de celle-ci. Le système gélifiant consiste en un mélange de cellulose microcristalline et de carboxyméthylcellulose. La thixotropie est forte, si bien qu'un gel selon ce document ne pourrait pas être distribué par la pompe doseuse d'un distributeur dans la mesure où le fort cisaillement liquéfierait la composition.

US-A- 4576645 concerne une composition gélifiée utilisable en confiserie ou comme support d'un principe actif dans un médicament. La composition consiste en un système émulsionné à viscosité élevée, qui peut le cas échéant être aéré. Selon ce document, la viscosité est donnée par l'association de gélifiant et d'émulsifiant, glycéride partiel du type de ceux utilisés dans la confection de suppositoires. L'alginate ou la gomme xanthane en tant que gélifiant ne jouent pas de rôle essentiel dans la thixotropie. Une telle émulsion ne serait pas utilisable dans un distributeur/doseur.

Le but de l'invention est de fournir une présentation sous forme de gel en distributeur avec élément doseur des principes actifs habituellement sous forme de sirop, comprimés ou capsules qui ne présente pas les inconvénients du gel dosable connu. On vise la commodité, l'hygiène et la sécurité d'utilisation, spécialement chez les enfants, bébés et nourrissons ainsi que chez toute personne ayant des problèmes de déglutition. De plus, dans un but particulier, on vise à autoriser une administration aux diabétiques. A ces objectifs s'ajoute la faculté de délivrer une dose quotidienne de faible volume en une ou deux pressions du doseur, ce qui implique dans certains cas une concentration élevée de principe actif en suspension dans le gel qui doit néanmoins présenter les propriétés de stabilité rhéologique et d'acceptabilité organoleptique compatibles avec le mode de distribution.

La demanderesse a trouvé une présentation commode, hygiénique et sure des principes actifs habituellement administrés sous forme de sirop remplissant les objectifs énoncés ci-dessus.

Ainsi, la composition pharmaceutique selon l'invention est caractérisée par le fait que le principe actif est réparti de manière homogène dans un gel hydrodispersible pseudoplastique ne coulant pas lors de la distribution et organoleptiquement acceptable, que le gel est contenu dans un emballage distributeur de volume intérieur 20 à 150 ml, muni d'une chambre de dosage de volume n'excédant pas 5 ml et d'une pompe doseuse adaptée pour distribuer une dose thérapeutique en une ou deux pressions par prise thérapeutique et que le gel contient 0,2 à 5% en poids d'un agent gélifiant choisi parmi les gommes xanthanes ou dextran, les celluloses et leurs dérivés, les carbomères, les acrylamides, les acrylamidines et les polyglycols.

Selon l'invention tout principe actif couramment administré par voie orale sous forme de sirop, comprimé ou capsule peut être présenté dans un gel en distributeur.

A titre d'exemple, on peut utiliser les principes actifs et leurs sels pharmaceutiquement acceptables dont la liste est donnée, dans les doses indiquées ci-après :

Les noms utilisés pour la dénomination des principes actifs sont les dénominations communes internationales.

Les doses sont exprimées en mg par prise thérapeutique. Une prise selon les cas correspond à une pression sur le distributeur soit 2 ml, parfois à 2 pressions, soit 4 ml.

La prise thérapeutique peut être renouvelée 2 à 5 fois par jour selon la prescription et le type de traitement.


- Antiacides par voie orale, à titre de pansements gastrointestinaux ou antiulcéreux :


| | |
|---|---|
| Phosphates d'aluminium ou de magnésium | 500-600 mg/4 ml |
| Hydroxyde d'aluminium et hydroxyde de magnésium | 400 mg/ 400 mg/4 ml |
| Sucralfate | 500-1000 mg/4 ml |

- Antidiarréhiques :

  Polyphénols insolubles de Caroube 500 mg/2 ml

  Lopéramide 1-4 mg/2 ml

- Antihistaminiques anti H1 :

  Carbinoxamine 2 mg/2 ml

  Acrivastine 1-10 mg/2 ml

  Tripolidine 1-100 mg/2 ml

- Anti nauséeux :

  Dimenhydrinate 10-150 mg/2 ml

- Antitussifs :

  Cloperastine 4-10 mg/2 ml

  Codéine 10-30 mg/2 ml

  Dextromethorphan 5-30 mg/2-4 ml

- Antiinflammatoires :

  Ibuprofène 100-600 mg/4 ml

  Flurbiprofène 25-300 mg/2-4 ml

  Diclofénac 10-150 mg/2-4 ml

- Antalgiques/antipyrétiques :

  Dextropropoxyphène 30-70 mg/2 ml

  Paracétamol 125-500 mg/2-4 ml

  Aspirine (sel) 50-500 mg/2-4 ml

- Mucomodificateurs bronchiques :

  Acétylcystéine (stabilisée) 100-600 mg/4 ml

| | |
|---|---|
| Carbocistéine | 100-750 mg/2-4 ml |
| Guaïfénésine | 50-200 mg/2-4 ml |
| Ambroxol | 3-30 mg/2-4 ml |

- Antispasmodiques :

| | |
|---|---|
| Phloroglucinol | 50-150 mg/2-4 ml |

- Analeptiques respiratoires/antiasthmatiques :

| | |
|---|---|
| Théophylline | 50-200 mg/2-4 ml |

- Alpha-sympathomimétiques systémiques :

| | |
|---|---|
| Pseudoéphédrine | 25-120 mg/2-4 ml |

- Vitamines et/ou oligoéléments sous forme de
  complexe vitaminé      50-350 mg/2-4 ml

- Laxatifs :

| | |
|---|---|
| Docusate | 20-200 mg/2-4 ml |
| Bisacodyl | 5-30 mg/2 ml |

On peut bien entendu utiliser des associations de principes actifs compatibles. La liste ci-après est donnée à titre indicatif :

- Alpha-sympathomimétique et antihistaminique anti
  H1 :

| | |
|---|---|
| Pseudoéphédrine et | 25-120 mg/ |
| Triprolidine | 1-100 mg/2-4 ml |

- Antihistaminique et antitussif opiacé :

| | |
|---|---|
| Pseudoéphédrine et | 25-120 mg/ |
| Dextrométhorphan | 5-30 mg/2-4 ml |

- Alpha-sympathomimétique et mucomodificateur bronchique :

  Pseudoéphédrine et                  25-120 mg/
  Guaïfénésine                        50-250 mg/2-4 ml

- Alpha-sympathomimétique, antitussif et antihista- minique :

  Pseudoéphédrine,                    25-120 mg/
  Dextrométhorphan et                  5-30  mg/
  Triprolidine                        1-100 mg/2-4 ml

- Alpha-sympathomimétique, mucomodificateur et anti- histaminique :

  Pseudoéphédrine,                    25-120 mg/
  Guaïfénésine et                     50-200 mg/
  Triprolidine                        1-100 mg/2-4 ml

- Alpha-sympathomimétique, antihistaminique et anti- tussif opiacé :

  Pseudoéphédrine,                    25-120 mg/
  Triprolidine et                      1-100 mg/
  Codéine phosphate                    3-50  mg/2-4 ml

- Antihistaminique et antitussif opiacé :

  Triprolidine et                      1-100 mg/
  Dextrométhorphan                     5-30  mg/2-4 ml

- Antihistaminique et analgésique-antipyrétique :

  Triprolidine et                      1-100 mg/
  Paracétamol                         125-250 mg/2-4 ml

  Triprolidine et                      1-100 mg/

6

Ibuprofène                        125-250 mg/2-4 ml

- Alpha-sympathomimétique et analgésique-antipyré
  tique :

Pseudoéphédrine et              10-120 mg/
Paracétamol                     125-250 mg/2-4 ml

Pseudoéphédrine et              10-120 mg/
Ibuprofène                      125-250 mg/2-4 ml

- Antihistaminique et alpha-sympathomimétique :

Acrivastine et                  1-10  mg/
Pseudoéphédrine                 10-120 mg/2-4 ml

- Antihistaminique, alpha-sympathomimétique et antal-
  gique-antipyrétique :

Acrivastine,                    1-10  mg/
Pseudoéphédrine et              10-120 mg/
Paracétamol                     125-250 mg/2-4 ml

- Antihistaminique, alpha-sympathomimétique et antitussif opiacé :

Acrivastine,                    1-10  mg/
Pseudoéphédrine et              10-120 mg/
Dextrométhorphan                5-30  mg/2-4 ml

- Antihistaminique, alpha-sympathomimétique et mucomodificateur :

Acrivastine,                    1-10  mg/
Pseudoéphédrine et              10-120 mg/
Guaïfénésine                    50-250 mg/2-4 ml

- Antihistaminique, alpha-sympathomimétique et anti-

inflammatoire (dérivés aryl carboxyliques) :

| | |
|---|---|
| **Acrivastine,** | **1-10  mg/** |
| **Pseudoéphédrine et** | **10-120 mg/** |
| **Ibuprofène** | **50-600 mg/2-4 ml** |

Un emballage distributeur convenable comprend une chambre de dosage et une pompe doseuse permettant de distribuer par pression sur une tête d'actionnement de la pompe, un volume exact de médicament prédéterminé par la chambre de dosage. De tels distributeurs sont couramment utilisés en cosmétique pour appliquer par exemple des crèmes. Ils peuvent être constitués par exemple d'un corps cylindrique rempli de produit, en matière plastique, en aluminium ou en verre, fermé au fond par un piston et muni à la tête d'une pompe doseuse et qui, actionné par pression sur la tête, aspire une dose de produit puis l'évacue par un bec formé dans cette tête.

En variante, le corps peut comprendre une membrane souple en forme de doigt contenant le produit et un gaz propulseur qui exerce une pression sur la membrane de manière à en évacuer une dose de produit par le bec lorsqu'une pression sur la tête ouvre une soupape d'admission du produit.

Un emballage distributeur peut contenir 20 à 150 ml et de préférence 20 à 100 ml de gel.

De préférence, la chambre de dosage a un volume utile d'environ 2 ml, correspondant à la dose unitaire à distribuer.

L'excipient est sous forme d'un gel hydrodispersible pseudoplastique et plus ou moins thixotrope. La pseudoplasticité visée correspond à une résistance du gel qui est plastique jusqu'à un certain seuil de cisaillement mais se casse au delà de ce seuil. La thixotropie s'entend comme la propriété qu'a le gel de devenir moins visqueux lorsqu'il subit un cisaillement constant (un frottement constant dans l'élément doseur lors de la distribution), mais de retrouver sa structure initiale après supression du cisaillement et un temps de repos suffisant. La consistance du gel doit être telle qu'il soit pompable, qu'il soit suffisamment déformable pour remplir exactement le volume de la chambre de dosage mais non filant et qu'il puisse être évacué de la chambre et rompu pour former une dose extrudable sans couler lors de la distribution. De plus, cette propriété de pseudoplasticité lui permet de pouvoir être déposé sur un support, par exemple une cuillère et adhérer suffisamment à celle-ci pour ne pas tomber même lorsque la cuillère est retournée. Par ailleurs, étant hydrodispersible, il n'adhère pas sur les muqueuses sans toutefois être destructuré dans la bouche, de manière à faciliter la déglutition.

Le gel constitue une matrice qui doit être aussi inerte que possible vis à vis du principe actif et de sa biodisponibilité. Les propriétés rhéologiques définies précédemment peuvent être obtenues au moyen des gélifiants, le cas échéant en combinaison avec des modificateurs de rhéologie appropriés conférant ces propriétés à la matrice et les maintenant au cours du temps. Ces gélifiants peuvent être d'origine naturelle, par exemple les gommes xanthanes ou dextran obtenues par fermentation, végétale, par exemple les celluloses et dérivés, ou d'origine synthétique, par exemple les carbomères, acrylamides, acrylamidines et polyglycols.

Dans certains cas, il peut s'avérer utile de modifier la rhéologie de certains de ces agents gélifiants, par exemple de manière à modifier la thixotropie de la matrice, en particulier de la renforcer, par exemple par addition d'aluminosilicate de magnésium à une cellulose.

Bien entendu, la pseudoplasticité de la matrice doit être adaptée à la conception du distributeur, en particulier au type, par exemple pompe à piston ou à soufflet de son élément doseur.

L'agent gélifiant représente 0,2 à 5% en poids de la composition.

Selon la nécessité thérapeutique, le principe actif peut être présent dans la matrice gel sous forme de solution homogène, par exemple lorsqu'il est très hydrosoluble ou lorsque la dose thérapeutique est faible ou sous forme de dispersion. Dans certains cas, le principe actif doit subir certaines opérations préalablement à sa dispersion dans la matrice gel, destinées à pouvoir augmenter sa concentration ou à masquer son goût, par exemple dans le cas où il est amer. On peut envisager de le solubiliser dans un solvant qui est inerte vis à vis des constituants de la matrice gel, puis de l'émulsionner, par exemple en le dissolvant dans un lipide, puis en réalisant une émulsion huile dans l'eau, c'est-à-dire en dispersant les gouttes d'huile dans le gel. On peut le disperser sous forme de microcristaux. On peut l'encapsuler dans un système ouvert, newtonien, par exemple une microsponge, comme par exemple un adsorbat solide poreux micronisé à base de trisilicate d'aluminium ou brownien, par exemple la béta-cyclodextrine, ou dans un système fermé, newtonien, matriciel, par exemple des microsphères ou vésiculaire, par exemple des microcapsules ou dans un système fermé brownien, matriciel, par exemple des nanocapsules ou vésiculaire, par exemple des nanocapsules de synthèse ou des liposomes. On peut également l'enrober par exemple par coacervation, coprécipitation ou polymérisation interfaciale. Ces techniques peuvent être mises en oeuvre par lit d'air fluidisé, par séchage, par pulvérisation ou par évaporation des solvants non miscibles en émulsion.

La composition peut également contenir des agents sucrants ou édulcorants, conservateurs, solubilisants, aromatisants et colorants. Lorsque le principe actif est amer, son amertume peut être masquée par addition d'agents sucrant, à condition toutefois que l'agent sucrant en question ne modifie pas notablement les propriétés rhéologiques de la matrice gel.

A titre d'agent sucrant, on peut utiliser par exemple le glucose et ses polymères, de préférence le saccharose, de dernier à une concentration de 20 à 30% en poids de la composition, en quantité suffisante, > à 20% pour masquer, au moins partiellement l'amertume. A une concentration supérieure à 30%, le saccharose conférerait des propriétés filantes au gel qui perdrait sa pseudoplasticité. On peut utiliser d'autres agents édulcorants renforçateurs du pouvoir sucrant du saccharose, par exemple le cyclamate de sodium et/ou de préférence le glycyrrhizinate d'ammonium à une concentration de 0,01 à 0,6% en poids de la composition.

Dans un mode de réalisation particulier de la composition pharmaceutique permettant l'administration des principes actifs aux diabétiques, l'agent édulcorant peut être par exemple l'aspartame à la concentration de 0,03 à 0,6% en poids de la composition. On peut ajouter à la composition un agent solubilisant du principe actif, par exemple le glycérol.

L'invention concerne également un procédé de préparation d'une composition pharmaceutique emballée, caractérisé par le fait que l'on dissout ou disperse le principe actif dans un milieu aqueux, que l'on mélange la solution ou la dispersion avec des agents sucrant, conservateur et aromatisant, puis que l'on ajoute un agent gélifiant au mélange obtenu sous agitation et que l'on conditionne le gel sirop ainsi formé dans un emballage distributeur muni d'une chambre de dosage et d'une pompe doseuse.

Dans un mode de réalisation particulier du procédé selon l'invention, on inclut le principe actif dans un système capsulaire en vue de modifier les caractéristiques organoleptiques de la composition préalablement à sa dissolution ou dispersion dans le milieu aqueux.

De préférence, le produit est exempt d'air, ce qui peut être réalisé par exemple par mélange des ingrédients sous vide. L'absence d'air permet de produire un gel à densité contrôlée et d'améliorer la conservation de la composition du fait de l'inclusion d'un minimum d'oxygène.

Les exemples ci-après illustrent l'invention. Dans ceux-ci, les parties et pourcentages sont pondéraux sauf indication contraire.

## Exemple 1

A 84,09 kg d'eau distillée à 60°C on ajoute sous brassage rapide 0,2 kg de bromhydrate de dextrométhorphan, puis 1,6 kg de béta-cyclodextrine. Après refroidissement à 20°C, on ajoute successivement à la solution 10 kg de glycérol puis 0,15 kg d'aspartame, 0,2 kg de benzoate de sodium, 0,15 kg d'acide citrique jusqu'à obtenir un pH de 4-4,5, 0,2 kg d'arôme de banane et 0,01 kg de colorant rouge, sous agitation en prenant soin de dissoudre chaque composant avant d'ajouter le suivant. Les opérations de mélange s'effectuent dans un réacteur mis préalablement sous vide. On ajoute alors sous agitation lente 2,5 kg de gomme xanthane sous forme de granulés au sirop obtenu. On conditionne ensuite le gel-sirop obtenu dans des emballages distributeurs cylindriques à pompe doseuse (vario dispenser®) en matière plastique de 75 ml se présentant la tête en bas que l'on ferme avec un piston servant de fond après avoir dégazé le cylindre rempli.

Par pression sur la tête de distribution, on verse une dose exacte de 2 ml de gel-sirop dans une cuillère que l'on peut retourner sans que le produit ne coule.

## Exemples 2-9

On fabrique comme à l'exemple 1 des gels-sirops dont la composition est indiquée dans le tableau I ci-après.

Dans certains cas seulement, le principe actif est adsorbé sur de la béta-cyclodextrine (exemples 4,5,6 et 7).

A l'exemple 9, le principe actif est mis en contact avec du trisilicate de magnésium à titre d'adsorbat solide poreux micronisé.

TABLEAU I

| Exemple % | 2 | 3 | 4 | 5 | 6 | 7 | 8 | 9 |
|---|---|---|---|---|---|---|---|---|
| Bromhydrate de dextrométhorphan | 0,2 | 0,2 | 0,2 | 0,2 | 0,2 | 0,4 | 0,2 | 2 |
| Beta-cyclodextrine | -- | -- | 1,6 | 1,6 | 1,6 | 3,6 | -- | -- |
| Adsorbat solide micronisé | -- | -- | -- | -- | -- | -- | -- | 18 |
| Saccharose | 30 | 30 | -- | -- | -- | -- | 30 | -- |
| Glycérol | -- | -- | 10 | 10 | 10 | -- | -- | -- |
| Monoammonium glycyrrhizinate | 0,1 | 0,1 | -- | -- | -- | -- | 0,1 | -- |
| Aspartame | -- | -- | 0,15 | 0,15 | 0,15 | 0,15 | -- | 0,2 |
| Benzoate de sodium | 0,3 | 0,3 | 0,2 | 0,2 | 0,2 | 0,3 | 0,3 | 0,2 |
| Parabenzoate de méthyle | 0,02 | 0,02 | -- | -- | -- | 0,02 | 0,02 | -- |
| Acide citrique | -- | -- | 0,15 | 0,15 | 0,15 | -- | -- | 0,15 |
| Arôme | 0,02 chocolat | 0,02 fraise | 0,2 abricot | 0,2 fraise | 0,2 /banane-menthe | 0,04 fraise | 0,02 fraise | 0,2 fraise |
| Colorant | -- | -- | -- | -- | 0,03 | 0,02 | -- | 0,03 |
| Gomme xanthane | 2 | 2,2 | 2,5 | 2,5 | 2,5 | 2,5 | 1,9 | 1,5 |
| Hydroxypropyl-méthylcellulose | -- | -- | -- | -- | -- | -- | 0,1 | -- |
| Eau | 67,36 | 67,14 | 85,1 | 85,1 | 84,97 | 92,97 | 67,36 | 77,72 |

Tous les gels-sirops précédents peuvent être versés dans une cuillère en une dose exacte sans que le produit ne coule, même en retournant la cuillère.

Exemple 10-12

On fabrique des gels-sirops comme à l'exemple 1 en utilisant comme agent gélifiant l'hydroxyéthylcellulose en combinaison avec un silicate comme modificateur de thixotropie et en mettant en oeuvre le paracétamol à titre de principe actif.

La composition des gels-sirops est indiquée dans le tableau II ci-après.

## TABLEAU II

| Exemple % | 10 | 11 | 12 |
|---|---|---|---|
| Paracétamol | 3 | 6 | 12,5 * |
| Hydroxyéthylcellulose | 2 | 2 | 2 |
| Silicate de magnésium et d'aluminium | 1 | 1 | 1 |
| Glycérol | 15 | 15 | 15 |
| Sorbitol | 15 | 15 | 15 |
| Méthylparaben | 0,15 | 0,15 | 0,15 |
| Saccharinate de sodium | 0,1 | 0,1 | 0,1 |
| Monoammonium glycyrrhizinate | 0,03 | 0,03 | 0,03 |
| Cyclamate de sodium ou de calcium | 0,03 | 0,03 | 0,03 |
| Arôme d'abricot | 0,35 | 0,35 | 0,35 |
| Colorant | q.s. | q.s. | q.s. |
| Eau | q.s.p. | q.s.p. | q.s.p. |

Légende :

\* = Dans l'exemple 12, le paracétamol est sous forme de poudre enrobée en lit fluidisé, le % indiqué étant relatif au principe actif.

q.s. = quantité souhaitée

q.s.p. = complément à 100.

Les gels-sirops précédents ont un aspect, une stabilité et un goût tout à fait acceptables. Par ailleurs, ils peuvent être dosés exactement et distribués sans couler.

Exemples 13-20

Dans ces exemples, on prépare des gels-sirops au dextromethorphan comme à l'exemple 1, en utilisant toutefois les différents agents gélifiants mentionnés, dans les proportions indiquées dans le tableau III ci-

après.

TABLEAU III

| Exemple % | 13 | 14 | 15 | 16 | 17 | 18 | 19 | 20 |
|---|---|---|---|---|---|---|---|---|
| Dextrométhorphan.HBr | 0,78 | 0,78 | 0,78 | 0,78 | 0,78 | 0,78 | 0,78 | 0,78 |
| Beta-cyclodextrine | 12 | 12 | 12 | 12 | 12 | 12 | 12 | 12 |
| Carboxyméthylcellulose | -- | -- | -- | -- | -- | -- | 2 | 1 |
| Hydroxyéthylcellulose | -- | 1 | -- | -- | -- | -- | -- | -- |
| Hydroxypropylcellulose 1 | 2 | -- | 2 | -- | -- | -- | -- | -- |
| Hydroxypropylcellulose 2 | -- | -- | -- | 2,5 | -- | -- | -- | -- |
| Hydroxypropylméthyl-cellulose 3 | -- | -- | -- | -- | -- | 2,5 | -- | -- |
| Hydroxypropylméthyl-cellulose 4 | -- | -- | -- | -- | 2,5 | -- | -- | -- |
| Silicate de magnésium et d'aluminium | 1 | 1 | 1 | 1 | 1 | 1 | 1 | 1 |
| Glycérol | 15 | 15 | 15 | 15 | 15 | 15 | 15 | 15 |
| Sorbitol | 15 | 15 | -- | -- | -- | -- | -- | -- |
| Méthylparaben | 0,15 | 0,15 | 0,15 | 0,15 | 0,15 | 0,15 | 0,15 | 0,15 |
| Saccharinate de sodium | 0,1 | 0,1 | 0,1 | 0,1 | 0,1 | 0,1 | 0,1 | 0,1 |
| Monoammonium glycyrrhi-zinate | 0,03 | 0,03 | 0,03 | 0,03 | 0,03 | 0,03 | 0,03 | 0,03 |
| Cyclamate de sodium ou de calcium | 0,03 | 0,03 | 0,03 | 0,03 | 0,03 | 0,03 | 0,03 | 0,03 |
| Arôme d'abricot | 0,35 | 0,35 | 0,35 | 0,35 | 0,35 | 0,35 | 0,35 | 0,35 |
| Colorant | q.s. | q.s. | q.s. | q.s. | q.s. | q.s. | q.s. | q.s. |
| Eau | q.s.p. | q.s.p. | q.s.p. | q.s.p. | q.s.p. | q.s.p. | q.s.p. | q.s.p. |

Légende :

1 = Klucel HF ®; 2 = Klucel MF ®; 3 = Methocel K100M Prem. ®; 4 = Methocel 4M ®

Les gels-sirops précédents ont un aspect, une stabilité et un goût tout à fait acceptables. Par ailleurs, ils peuvent être dosés exactement et distribués sans couler.

Exemples 21-27

On prépare les gels-sirops comme à l'exemple 1, en utilisant toutefois différents principes actifs et différents agents gélifiants dans les proportions indiquées au tableau IV ci-après.

<div align="center">TABLEAU IV</div>

| Exemple<br>% | 21 | 22 | 23 | 24 | 25 | 26 | 27 |
|---|---|---|---|---|---|---|---|
| Paracétamol | 12,5* | -- | -- | 12,5* | -- | 3 | 2,72 |
| Ibuprofen | -- | 10* | -- | -- | -- | -- | -- |
| Phenyléphrine HCL | -- | -- | 0,5 | -- | -- | -- | -- |
| Chlorphénamine "Maleas" | -- | -- | 0,2 | -- | -- | -- | -- |
| Pseudoéphédrine.HCl | -- | -- | -- | -- | -- | -- | 0,54 |
| Triprolidine.HCl | -- | -- | -- | -- | -- | -- | 0,03 |
| Dextrométhorphan.HBr | -- | -- | -- | -- | 0,78 | -- | -- |
| Beta-cyclodextrine | -- | -- | -- | -- | 12 | -- | -- |
| Hydroxyéthylcellulose | 1,5 | 1,5 | -- | -- | -- | -- | -- |
| Gomme xanthane | -- | -- | 2,5 | -- | -- | 2,2 | 2,2 |
| Carbomère 934P | -- | -- | -- | 1 | 1 | -- | -- |
| Glycérol | 15 | 15 | 15 | 15 | 15 | 10 | 15 |
| Propylèneglycol | -- | 10 | -- | -- | -- | -- | 8,65 |
| Sorbitol | 15 | -- | -- | 15 | 15 | 15 | 15 |
| Ethanol à 95° | -- | -- | -- | -- | -- | -- | 8 |
| Benzoate de sodium | 0,2 | 0,2 | 0,2 | -- | -- | -- | -- |
| Méthylparaben | -- | -- | -- | 0,15 | 0,15 | 0,1 | 0,1 |
| Propylparaben | -- | -- | -- | -- | -- | 0,02 | -- |
| Saccharinate de sodium | 0,2 | 0,2 | 0,2 | 0,2 | 0,2 | 0,1 | 0,2 |
| Monoammonium glycyrrhizinate | 0,08 | 0,08 | 0,09 | 0,08 | 0,08 | -- | -- |
| Cyclamate de sodium ou de calcium | 0,02 | 0,02 | 0,03 | 0,02 | 0,02 | -- | -- |
| Arôme d'abricot | 0,35 | 0,35 | 0,35 | 0,35 | 0,35 | 0,35 | -- |
| Arôme de framboise | -- | -- | -- | -- | -- | 0,3 | -- |
| Arôme banane-vanille | -- | -- | -- | -- | -- | -- | 0,2 |
| Colorant | q.s. | q.s. | q.s. | q.s. | q.s. | q.s. | q.s. q.s. |
| Eau | q.s.p. | q.s.p. | q.s.p. | q.s.p. | q.s.p. | q.s.p. | q.s.p. q.s.p. |

Légende :

\* = le paracétamol (exemples 21 et 24) et l'ibuprofen (exemple 22) sont sous forme de poudre enrobée en lit fluidisé, le % indiqué étant relatif au principe actif.

les gels-sirops précédents ont un aspect, une stabilité et un goût tout à fait acceptables. Ils peuvent être dosés exactement et distribués sans couler.

Exemples 28-41

On prépare des gels-sirops comme à l'exemple 1, en utilisant les principes actifs et les agents gélifiants dans les proportions indiquées au tableau V ci-après.

TABLEAU V

| Exemple % | 28 | 29 | 30 | 31 | 32 | 33 | 34 |
|---|---|---|---|---|---|---|---|
| Paracétamol | -- | -- | -- | 3,12* | 3,12* | 12,5* | 6,25* |
| Ibuprofen | -- | 6,66* | -- | -- | -- | -- | -- |
| Carbonate de la Dihydroxyaluminium | 8,75 | -- | -- | -- | -- | -- | -- |
| Aminoacétate | 3,75 | -- | -- | -- | -- | -- | -- |
| Pseudoéphédrine.HCl | -- | -- | 0,75 | 0,63 | 0,63 | -- | 0,75 |
| Triprolidine.HCl | -- | -- | 0,03 | 0,03 | 0,03 | -- | -- |
| Mix polyvitaminé | -- | -- | -- | -- | -- | -- | -- |
| Guaïfenesin | -- | -- | -- | -- | -- | -- | -- |
| Acrivastine | -- | -- | -- | -- | -- | -- | 0,08 |
| Carbocystéine | -- | -- | -- | -- | -- | -- | -- |
| Dextromethorphan.HBr | -- | -- | 0,37 | -- | -- | -- | -- |
| Béta-cyclodextrine | -- | -- | 5,2 | -- | -- | -- | -- |
| Gomme xanthane | 2,3 | 2,3 | 2,5 | -- | -- | -- | 2,3 |
| Carbomère 934P | -- | -- | -- | 1,7 | -- | -- | -- |
| Méthylcellulose | -- | -- | -- | -- | 2,74 | -- | -- |
| Hydroxypropyl-méthylcellulose | -- | -- | -- | -- | -- | 2,48 | -- |
| Glycérol | 15 | 15 | 10 | -- | -- | -- | 15 |
| Propylèneglycol | -- | 6,66 | -- | -- | -- | -- | -- |
| Hydroxyde de sodium | -- | -- | -- | 0,79 | -- | -- | -- |
| Benzoate de sodium | 0,2 | 0,2 | 0,2 | 0,2 | 0,2 | 0,2 | 0,2 |
| Saccharinate de sodium | 0,2 | 0,2 | 0,15 | 0,2 | 0,2 | 0,2 | 0,2 |
| Monoamonium glycyrrhizinate | 0,03 | 0,03 | 0,03 | 0,03 | 0,03 | 0,03 | 0,03 |
| Cyclamate de sodium ou de calcium | 0,03 | 0,03 | 0,03 | 0,03 | 0,03 | 0,03 | 0,03 |
| Arôme d'abricot | 0,35 | 0,35 | 0,35 | 0,35 | 0,35 | 0,35 | 0,35 |
| Colorant | q.s. | q.s. | q.s. | q.s. | q.s. | q.s. | q.s. q.s. |
| Eau | q.s.p. | q.s.p. | q.s.p. | q.s.p. | q.s.p. | q.s.p. | q.s.p. q.s.p. |

TABLEAU V (Suite)

| Exemple % | 35 | 36 | 37 | 38 | 39 | 40 | 41 |
|---|---|---|---|---|---|---|---|
| Paracétamol | -- | -- | -- | -- | -- | -- | -- |
| Ibuprofen | -- | -- | -- | 5* | 5* | 5* | -- |
| Carbonate de la Dihydroxyaluminium Aminoacétate | -- | -- | -- | -- | -- | -- | -- |
| Pseudoéphédrine.HCl | 0,75 | 0,75 | -- | 0,75 | -- | -- | -- |
| Triprolidine.HCl | -- | -- | 0,03 | -- | -- | -- | -- |
| Mix polyvitaminé | -- | -- | -- | -- | -- | -- | 3,5 |
| Guaïfenesin | -- | 5 | -- | -- | -- | -- | -- |
| Acrivastine | 0,08 | 0,08 | -- | -- | -- | -- | -- |
| Carbocystéine | -- | -- | -- | -- | -- | 19 | -- |
| Dextromethorphan.HBr | 0,37 | -- | -- | -- | -- | -- | -- |
| Béta-cyclodextrine | 5,2 | -- | -- | -- | -- | -- | -- |
| Gomme xanthane | 2,3 | 2,3 | 2,3 | 2,3 | 2,3 | 2,3 | 2,3 |
| Carbomère 934P | -- | -- | -- | -- | -- | -- | -- |
| Méthylcellulose | -- | -- | -- | -- | -- | -- | -- |
| Hydroxypropyl-méthylcellulose | -- | -- | -- | -- | -- | -- | -- |
| Glycérol | 15 | 15 | 15 | 15 | 15 | 15 | 15 |
| Propylèneglycol | -- | -- | -- | -- | -- | -- | -- |
| Hydroxyde de sodium | -- | -- | -- | -- | -- | -- | -- |
| Benzoate de sodium | 0,2 | 0,2 | 0,2 | 0,2 | 0,2 | 0,2 | 0,2 |
| Saccharinate de sodium | 0,2 | 0,2 | 0,2 | 0,2 | 0,2 | 0,2 | 0,2 |
| Monoamonium glycyrrhizinate | 0,03 | 0,03 | 0,03 | 0,03 | 0,03 | 0,03 | 0,03 |
| Cyclamate de sodium ou de calcium | 0,03 | 0,03 | 0,03 | 0,03 | 0,03 | 0,03 | 0,03 |
| Arôme d'abricot | 0,35 | 0,35 | 0,35 | 0,35 | 0,35 | 0,35 | 0,35 |
| Colorant | q.s. | q.s. | q.s. | q.s. | q.s. | q.s. | q.s. q.s. |
| Eau | q.s.p. | q.s.p. | q.s.p. | q.s.p. | q.s.p. | q.s.p. | q.s.p. q.s.p. |

<u>Légende</u> :

```
* =   le paracétamol (exemples 31,32,33 et 34) et l'ibu-
      profen (exemples 29,38,39 et 40) sont sous forme de
      poudre enrobée en lit fluidisé, le % indiqué étant
      relatif au principe actif.
```

Les gels-sirops précédents ont un aspect, une stabilité et un goût tout à fait acceptables. Ils peuvent être dosés exactement et distribués sans couler.

**Revendications**

1.  Composition pharmaceutique dans un emballage, dans laquelle la solution d'un principe actif est habituellement présentée dans un gel sirop administré par voie orale, caractérisée par le fait que le principe actif est réparti de manière homogène dans un gel hydrodispersible pseudoplastique ne coulant pas lors de la distribution et organoleptiquement acceptable, que le gel est contenu dans un emballage distributeur de volume intérieur 20 à 150 ml, muni d'une chambre de dosage de volume n'excédant pas 5 ml et d'une pompe doseuse adaptée pour distribuer une dose thérapeutique en une ou deux pressions par prise thérapeutique et que le gel contient 0,2 à 5% en poids d'un agent gélifiant choisi parmi les gommes xanthanes ou dextran, les celluloses et leurs dérivés, les carbomères, les acrylamides, les acrylamidines et les polyglycols.

2.  Composition selon la revendication 1, caractérisée par le fait que l'agent gélifiant est un mélange constitué d'un dérivé de cellulose et d'un aluminosilicate de magnésium à titre d'agent de thixotropie.

3.  Composition selon la revendication 1, caractérisée par le fait que le gel contient une gomme xanthane à titre d'agent gélifiant.

4.  Composition selon la revendication 1, caractérisée par le fait que le principe actif est inclus dans un système capsulaire ouvert ou fermé, matriciel ou vésiculaire en vue de modifier les caractéristiques organoleptiques de la composition.

5.  Composition selon la revendication 1, caractérisée par le fait que le principe actif est dispersé au sein d'une émulsion liquide/liquide de type huile dans l'eau ou dans une suspension solide/liquide.

6.  Composition selon la revendication 1, caractérisée par le fait qu'elle contient des agents sucrants ou édulcorants , conservateurs, colorants et aromatisants.

7.  Composition selon la revendication 1, caractérisé par le fait que le principe actif est un antiacide, antidiarrhéique, antihistaminique, antinauséeux, antitussif, antiinflammatoire, antalgique/antipyrétique, mucomodificateur bronchique, antispasmodique, analeptique respiratoire/antiasthmatique, alpha-sympathomimétique systémique, laxatif, un complexe vitaminé ou une combinaison compatible de ces principes actifs.

8.  Procédé de préparation d'une composition pharmaceutique emballée, caractérisé par le fait que l'on dissout ou disperse le principe actif dans un milieu aqueux, que l'on mélange la solution ou la dispersion avec des agents sucrant, conservateur et aromatisant, puis que l'on ajoute un agent gélifiant au mélange obtenu sous agitation et que l'on conditionne le gel sirop ainsi formé dans un emballage distributeur muni d'une chambre de dosage et d'une pompe doseuse, que l'agent gélifiant est présent à raison de 0,2 à 5% en poids et qu'il est choisi parmi les gommes xanthanes ou dextran, les celluloses et leurs dérivés, les carbomères, les acrylamides, les acrylamidines et les polyglycols.

9.  Procédé selon la revendication 8, caractérisé par le fait que, préalablement à sa dissolution ou dispersion dans le milieu aqueux, on inclut le principe actif dans un système capsulaire en vue de

EP 0 479 005 B1

modifier les caractéristiques organoleptiques de la composition.

**Claims**

1. A pharmaceutical composition in a pack, in which the solution of an active principle is normally presented as an orally administered gel syrup, characterized in that the active principle is homogeneously distributed in a pseudo-plastic water-dispersible gel which does not run during dispensing and is organoleptically acceptable, in that the gel is contained in a dispenser pack having an internal volume of 20 to 150 ml and provided with a metering compartment not exceeding 5 ml in volume and with a metering pump designed to dispense a therapeutic dose in one or two depressions per therapeutic dose and in that the gel contains 0.2 to 5% by weight of a gelling agent selected from xanthan gums or dextran, celluloses and their derivatives, carbomers, acrylamides, acrylamidines and polyglycols.

2. A composition as claimed in claim 1, characterized in that the gelling agent is a mixture consisting of a cellulose derivative and a magnesium alumosilicate as thixotropic agent.

3. A composition as claimed in claim 1, characterized in that the gel contains a xanthan gum as gelling agent.

4. A composition as claimed in claim 1, characterized in that the active principle is encapsulated in an open or closed matrix or vesicular system to modify the organoleptic characteristics of the composition.

5. A composition as claimed in claim 1, characterized in that the active principle is dispersed in a liquid/liquid emulsion of the oil-in-water type or in a solid/liquid suspension.

6. A composition as claimed in claim 1, characterized in that it contains sugars or sweetening agents, preservatives, colourants and flavourings.

7. A composition as claimed in claim 1, characterized in that the active principle is an antacid, antidiarrhoeic, antihistaminic, anti-emetic, antitussive, anti-inflammatory, analgesic/antipyretic, bronchial mucomodifier, antispasmodic, respiratory analeptic/antihistaminic, systemic alpha-sympathomimetic, laxative, a vitamin complex or a compatible combination of these active principles.

8. A process for the production of a packed pharmaceutical composition, characterized in that the active principle is dissolved or dispersed in an aqueous medium, the solution or the dispersion is mixed with a sweetening agent, a preservative and a flavouring, after which a gelling agent is added with stirring to the mixture obtained and the gel syrup thus formed is packed in a dispenser pack equipped with a metering compartment and a metering pump, in that the gelling agent is present in a quantity of 0.2 to 5% by weight and in that it is selected from xanthan gums or dextran, celluloses and their derivatives, carbomers, acrylamides, acrylamidines and polyglycols.

9. A process as claimed in claim 8, characterized in that, before it is dissolved or dispersed in the aqueous medium, the active principle is encapsulated to modify the organoleptic characteristics of the composition.

**Patentansprüche**

1. Verpackte pharmazeutische Zusammensetzung, in der die Lösung eines Wirkstoffs gewöhnlich in einem oral verabreichten Sirupgel dargeboten wird, dadurch gekennzeichnet, daß der Wirkstoff in einem wasserdispergierbaren pseudoplastischen Gel homogen verteilt ist, das bei der Abgabe nicht fließt und organoleptisch akzeptabel ist, daß das Gel in einem Spender mit einem Innenvolumen von 20 bis 150 ml enthalten ist, der mit einer Dosierkammer, deren Volumen 5 ml nicht überschreitet, und einer Dosierpumpe versehen ist, die so ausgelegt ist, daß sie eine therapeutische Dosis durch ein oder zwei Druckbetätigungen pro therapeutische Gabe abgeben kann, und daß das Gel 0,2 bis 5 Gew.-% eines Geliermittels enthält, das ausgewählt ist aus Xanthan- oder Dextrangummen, Zellulosen und ihren Derivaten, Carbomeren, Acrylamiden, Acrylamidinen und Polyglycolen.

18

2. Zusammensetzung nach Anspruch 1, dadurch gekennzeichnet, daß das Geliermittel eine Mischung ist, die aus einem Cellulosederivat und einem Magnesiumalumosilikat als Thixotropiemittel besteht.

3. Zusammensetzung nach Anspruch 1, dadurch gekennzeichnet, daß das Gel als Geliermittel einen Xanthangummi enthält.

4. Zusammensetzung nach Anspruch 1, dadurch gekennzeichnet, daß der Wirkstoff zum Zweck der Veränderung der organoleptischen Merkmale der Zusammensetzung in ein offenes oder geschlossenes vesikuläres oder matrixförmiges Kapselsystem eingeschlossen ist.

5. Zusammensetzung nach Anspruch 1, dadurch gekennzeichnet, daß der Wirkstoff in einer Flüssig-Flüssig-Dispersion vom Typ Öl in Wasser oder in einer Fest-Flüssig-Suspension verteilt ist.

6. Zusammensetzung nach Anspruch 1, dadurch gekennzeichnet, daß sie Zucker oder Süßungsmittel, Konservierungsmittel, Farbstoffe und Aromamittel enthält.

7. Zusammensetzung nach Anspruch 1, dadurch gekennzeichnet, daß der Wirkstoff ein Antazidum, ein Antidiarrhoikum, ein Antihistaminikum, ein Antiemetikum, ein Antitussivum, ein Antiphlogistikum, ein Antalgikum/Antipyretikum, ein Mukolytikum, ein Antispasmodikum, ein Atemanaleptikum/Antiasthmatikum, ein systemisches Alphasympathomimetikum, ein Laxativum, ein Vitaminkomplex oder eine kompatible Kombination dieser Wirkstoffe ist.

8. Verfahren zur Herstellung einer verpackten pharmazeutischen Zusammensetzung, dadurch gekennzeichnet, daß man den Wirkstoff in einem wässrigen Medium löst oder dispergiert, daß man die Lösung oder die Dispersion mit Süßmitteln, Konservierungsmitteln und Aromamitteln mischt, daß man dann der erhaltenen Mischung unter Rühren ein Geliermittel beigibt und daß man das so gebildete Sirupgel in einem Spender verpackt, der mit einer Dosierkammer und einer Dosierpumpe versehen ist, daß das Geliermittel in einem Anteil von 0,2 bis 5 Gew.-% vorliegt und daß es ausgewählt ist aus Xanthan- oder Dextrangummen, Cellulosen und ihren Derivaten, Carbomeren, Acrylamiden, Acrylamidinen und Polyglycolen.

9. Verfahren nach Ansnpruch 8, dadurch gekennzeichnet, daß man den Wirkstoff vor seiner Lösung oder Dispersion in dem wässrigen Medium zur Veränderung der organoleptischen Eigenschaften der Zusammensetzung in ein Kapselsystem einschließt.